# EUROPÄISCHE PATENTSCHRIFT

(11) **EP 0 850 923 B1**
(45) Veröffentlichungstag und Bekanntmachung des Hinweises auf die Patenterteilung: **21.02.2001**
(21) Anmeldenummer: 97122374.8
(22) Anmeldetag: 18.12.1997
(51) Int. Cl.: C07C 319/28

(54) **Verfahren zur Auftrennung des Produktgasgemisches der katalytischen Synthese von Methylmercaptan**
Process for the separation of the gas mixture from the catalytic synthesis of methyl mercaptan
Procédé de séparation du mélange gazeux provenant de la synthése catalytique du méthylmercaptan

(30) Priorität: 27.12.1996 DE 19654516
(43) Veröffentlichungstag der Anmeldung: 01.07.1998
(73) Patentinhaber: Degussa-Hüls Aktiengesellschaft, 60287 Frankfurt am Main (DE)
(72) Erfinder: Hofen, Willi, 63517 Rodenbach (DE); Böck, Wolfgang, Dr., 63505 Langenselbold (DE); Rautenberg, Stephan, Dr., 63457 Hanau (DE); Sauer, Jörg, Dr., 63517 Rodenbach (DE); Arntz, Dietrich, Dr., Mobile, AL 36695 (US); Goedecke, Ralf, Dr., 63517 Rodenbach (DE); Taugner, Wolfgang, 63674 Altenstadt (DE); Sonnenschein, Raymund, Dr., 63526 Erlensee (DE)

(56) Entgegenhaltungen:
- FR-A- 2 477 538

## Beschreibung

Die Erfindung betrifft ein Verfahren zur Auftrennung des mit einer Temperatur von 100 bis 150°C und mit einem Druck von 6 bis 12 bar anfallenden Produktgasgemisches der katalytischen Synthese von Methylmercaptan aus Schwefelwasserstoff und Methanol. Das Produktgasgemisch der Synthese enthält neben dem gewünschten Methylmercaptan das bei der Reaktion entstehende Wasser und als Nebenprodukte Dimethylsulfid, Dimethylether und geringe Mengen Polysulfide sowie nicht umgesetztes Methanol, überschüssigen Schwefelwasserstoff und die im Sinne der Reaktion inerten Gase Stickstoff, Kohlendioxid, Kohlenmonoxid und Wasserstoff. Die Auftrennung des Produktgasgemisches in seine Komponenten dient zur Gewinnung von Methylmercaptan und Dimethylsulfid, zur Ausschleusung von Wasser und Inertgasanteilen sowie zur Rückführung von nicht verbrauchtem Methanol und Schwefelwasserstoff in den Synthesereaktor.

Methylmercaptan ist ein industriell wichtiges Zwischenprodukt für die Synthese von Methionin sowie für die Herstellung von Dimethylsulfoxid und Dimethylsulfon. Es wird heute überwiegend aus Methanol und Schwefelwasserstoff durch Umsetzung an einem Katalysator aus Aluminiumoxid hergestellt. Die Synthese des Methylmercaptans erfolgt gewöhnlich in der Gasphase bei Temperaturen zwischen 300 und 500°C und bei Drucken zwischen 1 und 25 bar. Zur Erhöhung von Aktivität und Selektivität des Katalysators wird dieser üblicherweise mit Kaliumwolframat als Promoter belegt. Die Umsetzung von Schwefelwasserstoff und Methanol zu Methylmercaptan ist ein exothermer Prozeß, bei dem pro Kilomol umgesetztes Methanol 28.500 kJ frei werden.

Der Gesamtprozeß der Methylmercaptan-Herstellung kann in zwei Abschnitte unterteilt werden. Der erste Abschnitt umfaßt die Aufbereitung des Eduktgasgemisches und seine Umsetzung zu Methylmercaptan. Der zweite Abschnitt beinhaltet die Auftrennung des Produktgasgemisches zur Gewinnung von Methylmercaptan und Rückführung von nicht verbrauchtem Methanol und Schwefelwasserstoff sowie Entsorgung von Abwasser und Abgasen. Die vorliegende Erfindung befaßt sich mit Verbesserungen im zweiten Abschnitt des Herstellungsprozesses.

Die DE-PS 17 68 826 beschreibt ein Verfahren zur Auftrennung des Produktgasgemisches der Methylmercaptan-Synthese, bei dem das Produktgasgemisch bei einem Druck von höchstens 11 bar und einer Temperatur von 10 bis 140°C destilliert wird. Die gasförmige Phase dieser Destillation besteht im wesentlichen aus Schwefelwasserstoff, inerten Gasen, Dimethylsulfid und Methylmercaptan. Methylmercaptan und Dimethylsulfid werden im Gegenstrom mit Methanol aus der gasförmigen Phase ausgewaschen. Der verbleibende Schwefelwasserstoff und die inerten Gase werden als Kreisgas in den Synthesereaktor zurückgeführt. Das beladene Waschmethanol wird zusammen mit dem praktisch schwefelwasserstofffreien Sumpf der Destillation erneut destillativ aufgearbeitet und ebenfalls in den Herstellprozeß zurückgeführt.

Das nach diesem Verfahren erhältliche Methylmercaptan enthält als geringe Beimengungen noch maximal 0,015 Gew.-% Schwefelwasserstoff und maximal 0,15 Gew.-% Methanol.

Die inerten Gase werden zum Teil als Verunreinigungen mit dem Schwefelwasserstoff-Frischgas in den Herstellprozeß eingeschleppt. Zusätzlich bilden sich inerte Gase durch Zersetzung von Methanol im Synthesereaktor. Um ein Ansammeln der Inertgase im Prozeß zu vermeiden, wird gemäß der DE-PS 17 68 826 ein Teil des Schwefelwasserstoff-Kreisgases ausgeschleust und verbrannt. Hierbei geht dem Prozeß wertvolles Schwefelwasserstoffgas verloren. Das bei der Verbrennung gebildete Schwefeldioxid muß zur Erfüllung heutiger Rauchgasnormen aus dem Rauchgas entfernt werden.

Das bei der Synthese von Methylmercaptan gebildete Wasser wird nach Abtrennung aus dem Produktgasgemisch zum Brechen der Azeotrope Methylmercaptan/Methanol und Dimethylsulfid/Methanol verwendet. Das Wasser muß schließlich kontinuierlich aus dem Prozeß ausgeschleust werden. Ein wesentliches Problem ist dabei die möglichst vollständige Entfernung von Methylmercaptan, Dimethylsulfid und Polysulfiden aus dem Abwasser, um Geruchsbelästigungen und Umweltschäden zu vermeiden.

Gemäß der FR 2 477 538 durchläuft das aus dem Reaktor austretende Produktgasgemisch zwei Kondensationsstufen zur Kondensation des größeren Teils des Gasgemisches. Das Kondensat wird bei einem Druck von 7 bar und einer Temperatur von 30°C in einem Phasentrenngefäß zur Trennung in eine organische und eine wäßrige Phase gesammelt. Die nicht kondensierten schwefelwasserstoffreichen Gase werden mit Methanol gewaschen und dann zur Verbrennung aus dem Prozeß ausgeschleust. Die Waschflüssigkeit wird ebenfalls im Phasentrenngefäß gesammelt. Organische und wäßrige Phase aus dem Phasentrenngefäß werden separat in Destillationskolonnen weiter verarbeitet.

Ein Problem der aus dem Stand der Technik bekannten Verfahren ist die Tatsache, daß die Auftrennung des Produktgasgemisches in die einzelnen Stoffströme mit ungenügender Trennschärfe erfolgt. Dies hat zur Folge, daß zum Beispiel mit dem Schwefelwasserstoff-Kreisgas auch Methylmercaptan wieder in den Synthesereaktor zurückgeführt wird und die zur Verbrennung ausgeschleusten inerten Gase noch hohe Anteile von Schwefelwasserstoff, Methylmercaptan und Methanol enthalten. Auch das aus dem Prozeß auszuschleusende Wasser weist keine genügende Reinheit auf, um ohne weitere Behandlung entsorgt werden zu können. Ganz allgemein führt diese ungenügende Trennung der Stoffströme zu höheren Energiekosten, da größere Stoffströme im Kreis geführt werden müssen. Darüber hinaus entstehen hohe Kosten durch Verbrennen von wertvollen Einsatzstoffen (Schwefelwasserstoff, Methanol) und durch die dadurch erforderlich werdende Nachbehandlung der Rauchgase.

Aufgabe der vorliegenden Erfindung ist es daher, ein Verfahren zur Auftrennung des Produktgasgemisches der Methylmercaptan-Synthese anzugeben, welches sich durch eine verbesserte Auftrennung des Produktgasgemisches in die einzelnen Stoffströme auszeichnet.

Diese Aufgabe wird gelöst durch ein Verfahren zur Auftrennung des mit einer Temperatur von 100 bis 150°C und mit einem Druck von 6 bis 12 bar anfallenden Produktgasgemisches der katalytischen Synthese von Methylmercaptan aus Schwefelwasserstoff und Methanol in die Komponenten Methylmercaptan, Dimethylsulfid, Polysulfide, Wasser, Methanol, Schwefelwasserstoff und Inertgase enthaltend die folgenden Verfahrensschritte:
a) Auftrennen des Produktgasstromes in ein wäßriges, Methanol und Wasser enthaltendes, Kondensat und in ein organisches, Schwefelwasserstoff, Methylmercaptan und Dimethylsulfid enthaltendes, Kondensat sowie in einen Restgasstrom, welcher Schwefelwasserstoff und Methylmercaptan enthält, durch zweistufige Partialkondensation, wobei das wäßrige Kondensat bei Temperaturen zwischen 55 und 65°C und das organische Kondensat bei Temperaturen zwischen 15 und 30°C kondensiert wird,
b) Absorption von Methylmercaptan und Dimethylsulfid aus dem Restgasstrom in einer ersten Wäsche mit Methanol und Aufteilen des gewaschenen, schwefelwasserstoffreichen Gasstromes in einen Kreisgasstrom und einen Ausschleusstrom im Volumenverhältnis von 5 : 1 bis 20 : 1,
c) Absorption von Schwefelwasserstoff aus dem Ausschleusstrom in einer zweiten Wäsche mit Methanol und Entfernen des gereinigten Ausschleusstromes aus dem Verfahrensablauf, wobei für die zweite Wäsche frisches Methanol verwendet wird, welches nach Beladung mit Schwefelwasserstoff als Waschmittel für die erste Wäsche eingesetzt wird,
d) Destillieren des beladenen Waschmethanols sowie des wäßrigen und des organischen Kondensats zur Abtrennung von Schwefelwasserstoff als gasförmiges Kopfprodukt von den restlichen Komponenten des Produktgasgemisches, welche als flüssiges Rohprodukt im Destillationssumpf anfallen und Einspeisen des abgetrennten Schwefelwasserstoffgases in den Kreisgasstrom oder in den Restgasstrom und
e) Auftrennen des Rohproduktes mittels weiterer Destillationen in die Komponenten Methylmercaptan, Dimethylsulfid, Dimethylether, Polysulfide, Methanol und Wasser.

Das Produktgasgemisch der Methylmercaptan-Synthese verläßt den Reaktor mit einer Temperatur im Bereich von 340 bis 360°C und einem Druck von 6 bis 12 bar. Zur Auftrennung des Produktgasgemisches in seine einzelnen Komponenten wird es zunächst auf eine Temperatur von etwa 100 bis 150°C abgekühlt. Dies kann zum Beispiel wie in der DE-PS 17 68 826 beschrieben im Wärmetausch mit Schwefelwasserstoff geschehen. Eine weitere Möglichkeit besteht darin, den Wärmeinhalt des Produktgasgemisches zur Verdampfung von Methanol zu nutzen (siehe die parallele Patentanmeldung ... .. ...).

Nach erfolgter Abkühlung wird das Produktgasgemisch zur Auftrennung in seine Komponenten und zur Gewinnung von Methylmercaptan nach dem erfindungsgemäßen Verfahren weiterverarbeitet.

Zunächst wird der Produktgasstrom mittels zweistufiger Partialkondensation in ein wäßriges Kondensat und in ein organisches Kondensat sowie in einen Restgasstrom aufgeteilt. Das wäßrige Kondensat besteht im wesentlichen aus Methanol und Wasser. Die Hauptbestandteile des organischen Kondensats sind Schwefelwasserstoff, Methylmercaptan und Dimethylsulfid, während der Restgasstrom Schwefelwasserstoff und Methylmercaptan als Hauptkomponenten enthält. Das wäßrige Kondensat wird bei einer Temperatur im Bereich zwischen 55 und 65°C vom Produktgasstrom abgetrennt. Das organische Kondensat wird im Temperaturintervall zwischen 15 und 30°C erhalten.

Durch die große Temperaturdifferenz zwischen Kühlwasser und Kondensationstemperatur des wäßrigen Kondensats kann die Kondensationswärme von Wasser und Methanol mit relativ kleinen Wärmeaustauschflächen und geringen Mengen an Kühlwasser dem Produktgasstrom entzogen werden.

Der nach Kondensation von wäßrigem und organischem Kondensat verbleibende Restgasstrom wird zur Absorption von Methylmercaptan und Dimethylsulfid mit Methanol gewaschen. Der Restgasstrom enthält nach dieser ersten Methanolwäsche mehr als 90 Vol.-% Schwefelwasserstoff. Der restliche Volumenanteil besteht im wesentlichen aus den Inertgasen Kohlendioxid, Kohlenmonoxid, Wasserstoff und Stickstoff. Die tatsächliche Zusammensetzung des gewaschenen Restgasstromes hängt hauptsächlich von der Reinheit des verwendeten Schwefelwasserstoff-Frischgases ab, welches dem Gesamtprozeß der Methylmercaptan-Herstellung im ersten Verfahrensschritt zugeführt wird. Der Anteil der Inertgase aus der Zersetzung von Methanol bei der katalytischen Umsetzung zu Methylmercaptan kann nur im geringen Maße durch die Prozeßparameter bei der Umsetzung beeinflußt werden.

Um eine Ansammlung der Inertgase im Gesamtprozeß der Methylmercaptan-Herstellung zu vermeiden, müssen die Inertgase kontinuierlich aus dem Prozeß ausgeschleust werden. Hierzu wird der gewaschene Restgasstrom in einen Kreisgasstrom und einen Ausschleusstrom im Volumenverhältnis von 5 : 1 bis 20 : 1 aufgeteilt. Das Volumenverhältnis von 5 : 1 wird bei einem Schwefelwasserstoff-Frischgas mit hohen Inertgasgehalten angewendet. Bei hochreinem Schwefelwasserstoff-Frischgas kann das Volumenverhältnis auf 20 : 1 erhöht werden.

Der Kreisgasstrom wird in den ersten Verfahrensabschnitt zurückgeführt. Der Ausschleusstrom wird erfindungsgemäß durch eine zweite Wäsche mit Methanol von Schwefelwasserstoff befreit. Nach der Wäsche enthält der gereinigte Ausschleusstrom weniger als 0,1 Vol.-% Schwefelwasserstoff und kann direkt einer Abgasverbrennung zugeführt werden. Das dabei entstehende Rauchgas kann unter Einhaltung der derzeit gültigen Rauchgasnormen direkt an die Atmosphäre abgegeben werden.

Diese Tatsache unterscheidet das erfindungsgemäße Verfahren wesentlich von den aus dem Stand der Technik bekannten Verfahren. Gemäß der FR 2 477 538 enthält der Ausschleusstrom mehr als 70 Vol.-% Schwefelwasserstoff. Ebenso besteht auch der Ausschleusstrom aus der DE-PS 17 68 826 überwiegend aus Schwefelwasserstoff. Dadurch geht den Verfahren aus dem Stand der Technik ein wertvoller Rohstoff verloren. Darüber hinaus macht der hohe Schwefelwasserstoffgehalt der Ausschleusgase bei einer eventuellen Verbrennung entsprechende Maßnahmen zur Entfernung von Schwefeldioxid aus den Rauchgasen notwendig.

Eine Besonderheit des erfindungsgemäßen Verfahrens ist die Tatsache, daß beide Methanolwäschen mit demselben Methanolstrom vorgenommen werden. Das frische Waschmethanol wird zunächst zur Wäsche des Ausschleusstromes verwendet. Dabei belädt sich das Waschmethanol mit Schwefelwasserstoff. Das beladene Waschmethanol wird dann zur Wäsche des Restgasstromes verwendet, wobei es das darin enthaltene Methylmercaptan und Dimethylsulfid absorbiert. Es hat sich gezeigt, daß die Vorbeladung des Waschmethanols mit Schwefelwasserstoff keine negativen Auswirkungen auf die Absorptionsfähigkeit für Methylmercaptan hat.

Bevorzugt wird die Absorption von Schwefelwasserstoff und Methylmercaptan aus dem Ausschleusstrom beziehungsweise dem Restgasstrom isotherm bei einer Temperatur im Bereich zwischen 20 und 30°C vorgenommen. Zu diesem Zweck muß die Absorptionswärme durch eine entsprechende Kühlung dem Waschmittelstrom entzogen werden.

Die hintereinandergeschaltete isotherme Absorption von Schwefelwasserstoff und Methylmercaptan führt zu erheblichen Einsparungen an Waschmethanol und liefert einen weitgehend schwefelwasserstofffreien Ausschleusstrom. Während in der DE-PS 17 68 826 pro Kilogramm Methylmercaptan 2,5 Kg Methanol zur Wäsche benötigt werden, kommt das erfindungsgemäße Verfahren mit einem Drittel dieser Menge aus.

Das Waschmethanol sowie die beiden Kondensate der Partialkondensation enthalten große Mengen an Schwefelwasserstoff (Waschmethanol: 15 - 20 Gew.-% H₂S; wäßriges Kondensat: 1 - 2 Gew.-%; organisches Kondensat: ca. 20 Gew.-% H₂S). Zur Abtrennung des Schwefelwasserstoffes werden die drei Stoffströme gemeinsam destilliert, wobei Schwefelwasserstoff als gasförmiges Kopfprodukt von den restlichen Komponenten des Produktgasgemisches abgetrennt wird. Der abgetrennte Schwefelwasserstoff wird in den Kreisgasstrom eingespeist. Neben Schwefelwasserstoff kann das gasförmige Kopfprodukt noch erhebliche Mengen Methylmercaptan enthalten. Dies führt dazu, daß das in den ersten Verfahrensabschnitt zurückgeführte Kreisgas bis zu 3 Vol.-% aus Methylmercaptan besteht. Dieser Gehalt an Methylmercaptan kann unter 1 Vol.-% verringert werden, wenn das gasförmige Kopfprodukt nicht direkt in den Kreisgasstrom eingespeist, sondern gemeinsam mit dem Restgasstrom der schon beschriebenen ersten Methanolwäsche zugeführt wird.

Das flüssige Sumpfprodukt, beziehungsweise Rohprodukt, der Schwefelwasserstoff-Abtrennung, wird zur Gewinnung von Methylmercaptan mittels weiterer Destillationen in die Komponenten Methylmercaptan, Dimethylsulfid, Dimethylether, Polysulfide, Methanol und Wasser aufgetrennt.

Bevorzugt werden vom Rohprodukt zunächst Methylmercaptan und die Nebenprodukte Dimethylsulfid und Dimethylether von Methanol und Wasser abgetrennt. Wegen der im Rohprodukt vorliegenden azeotropen Mischungen aus Methylmercaptan/Methanol sowie aus Dimethylsulfid/Methanol wird zur Brechung der Azeotrope eine extraktive Destillation angewendet, wobei Wasser als Extraktionsmittel dient. Hierbei fallen Methylmercaptan und Dimethylsulfid als Kopfprodukt an. Methanol und Wasser bilden den Destillationsrückstand. Durch das Extraktionswasser wird erreicht, daß kein Methanol azeotrop ins Kopfprodukt übergeht. Darüber hinaus hat sich gezeigt, daß kein zusätzlicher Rücklauf zur Verstärkung des Kopfproduktes benötigt wird. Dieses kann vielmehr ohne Zwischenkondensation direkt der weiteren Isolierung von Methylmercaptan zugeführt werden. Als Rücklauf bei der extraktiven Destillation dient das Extraktionswasser.

Zur Isolierung von Methylmercaptan kann das Kopfprodukt der extraktiven Destillation in einer weiteren Destillation in ein Methylmercaptan und Wasser enthaltendes Kopfkondensat und ein im wesentlichen aus Dimethylsulfid bestehendes Bodenprodukt aufgetrennt werden. Das Kopfkondensat wird durch eine flüssig-flüssig Phasentrennung in Methylmercaptan hoher Reinheit und Wasser aufgespalten.

Der Destillationsrückstand aus der extraktiven Destillation enthält zu etwa gleichen Anteilen Methanol und Wasser. Darüber hinaus enthält der Destillationsrückstand noch geringe Anteile von Leichtsiedern (Methylmercaptan, Dimethylether) sowie Dimethylsulfid und Polysulfide. Zur Wiedergewinnung von Methanol und Rückführung in den Gesamtprozeß kann der Destillationsrückstand erneut destilliert werden. Dabei wird Methanol am Kopf kondensiert und als Waschmethanol und für die Methylmercaptansynthese wieder verwendet. Wasser und Polysulfide fallen als Bodenprodukt an.

Bevorzugt wird ein Teil dieses Prozeßwassers (etwa 2/3 des Bodenproduktes) als Extraktionswasser in der extraktiven Destillation wieder verwendet. Das restliche Drittel, welches dem bei der Umsetzung gebildeten Reaktionswasser entspricht, wird aus dem Prozeß ausgeschleust. Um eine Anreicherung der Polysulfide im Prozeß zu verhindern und die Geruchsbelastung des Ausschleuswassers zu vermindern, werden die Polysulfide aus dem Prozeßwasser mit Dampf ausgetrieben und einer Abgasverbrennung zugeführt.

Die Auftrennung des Produktgasgemisches nach dem erfindungsgemäßen Verfahren wird zur Begrenzung der zur Kondensation der verschiedenen Komponenten benötigten Kühlleistungen bei Drücken von 1 bis 10 bar durchgeführt. In einer ersten Verfahrensstufe, die die oben beschriebenen Verfahrensschritte a) bis d) umfaßt, wird bei einer Druckhaltung von 6 bis 10 bar, bevorzugt 8 bar, gearbeitet. Die weitere Auftrennung des Rohproduktes aus der Schwefelwasserstoff-Abtrennung durch extraktive Destillation und nachfolgender Trennung in Methylmercaptan und Dimethylsulfid erfolgt bei Drucken von 4 bis 8 bar, bevorzugt bei 6 bar. Die Trennung von Methanol und Prozeßwasser wird schließlich bevorzugt bei Normaldruck von 1 bar vorgenommen.

Das Verfahren gemäß der Erfindung wird anhand der beiden Figuren beispielhaft erläutert. Es zeigen
- Figur 1:: Verfahrensschema für den Ersten Verfahrens abschnitt der Methylmercaptan-Herstellung
- Figur 2:: Verfahrensschema gemäß der Erfindung für den zweiten Verfahrensabschnitt der Methylmercaptan-Herstellung.

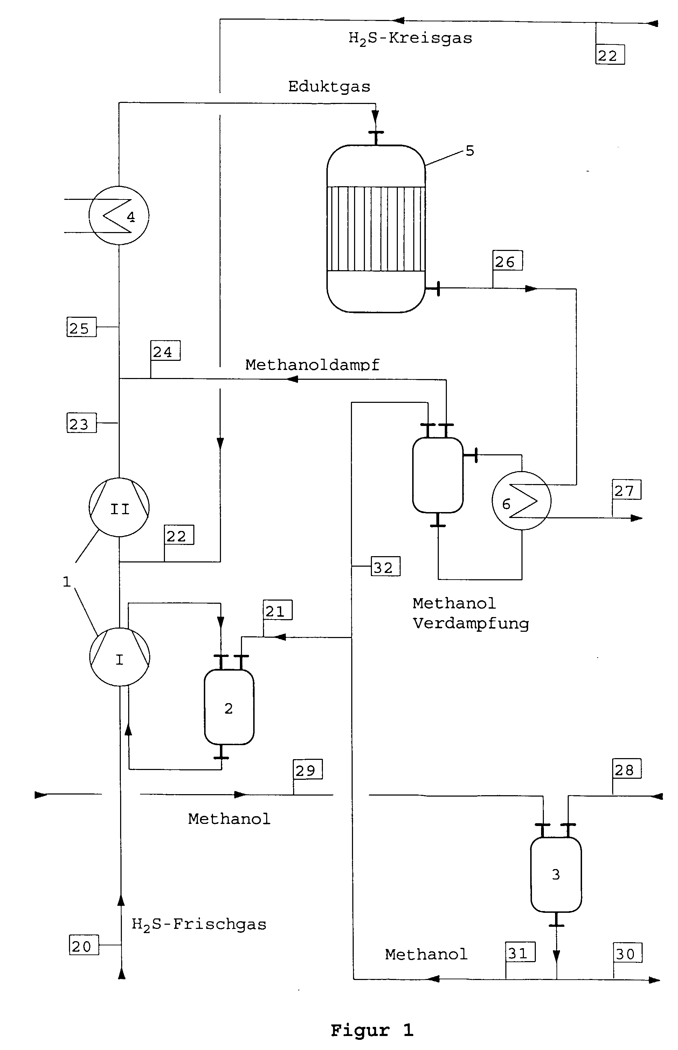

Figur 1 zeigt ein mögliches Verfahrensschema für den ersten Verfahrensabschnitt der Methylmercaptan-Herstellung. Es entspricht dem Verfahrensschema von Figur 1 aus der parallelen Patentanmeldung ... .. ... Im Reaktor 5 wird das Eduktgasgemisch 25 aus Schwefelwasserstoff und Methanol mit einem Molverhältnis von Schwefelwasserstoff zu Methanol von 1,8 an einem Aluminiumoxid-Katalysator bei einem Betriebsdruck von 10 bar und einer Reaktionstemperatur von 360°C zu Methylmercaptan und Nebenprodukten (Dimethylsulfid, Dimethylether, Polysulfide) umgesetzt. Der Katalysator ist zur Erhöhung seiner Aktivität und Selektivität gemäß Beispiel 2 aus der deutschen Patentanmeldung DE 196 39 584 mit 25 Gew.-% Cäsiumwolframat belegt.

Das Produktgasgemisch 26 wird nach Abkühlung auf 130°C als Produktstrom 27 an den zweiten Verfahrensabschnitt der Methylmercaptan-Herstellung übergeben. Der Wärmeinhalt des heißen Produktgasgemisches wird im Wärmetauscher 6 genutzt, um einen Teil des für die katalytische Umsetzung benötigten Methanols zu verdampfen.

Das für die Umsetzung benötigte Schwefelwasserstoff-Frischgas 20 wird mit Hilfe eines zweistufigen Schraubenverdichters 1 über einen Zwischendruck von 6 bar auf den Enddruck von 11 bar verdichtet. In diesem Beispiel wird hochreiner Schwefelwasserstoff mit einem Gehalt von mehr als 99 Gew.-% Schwefelwasserstoff verwendet. In die erste Stufe des Verdichters wird zur Temperaturbegrenzung flüssiges Methanol aus einem Pufferbehälter 2 eingespritzt. Ein Teil des Methanols wird durch die Verdichtungswärme der ersten Stufe verdampft. Der nicht verdampfte Anteil wird über den Pufferbehälter 2 im Kreis geführt. Verdampftes Methanol wird durch den Methanolstrom 21 aus dem Pufferbehälter 3 ersetzt. Nach der ersten Verdichtungsstufe wird dem Schwefelwasserstoff/Methanol Gasgemisch das aus dem zweiten Verfahrensabschnitt zurückgeführte Schwefelwasserstoff-Kreisgas 22 zugemischt. Dem verdichteten Gasgemisch 23 wird zur Einstellung das Molverhältniss von Schwefelwasserstoff/Methanol Methanoldampf 24 zugemischt. Das so erhaltene Eduktgasgemisch 25 wird im Gaserhitzer 4 auf etwa 170°C erwärmt und dann mit einem Druck von 10 bar in den Reaktor eingeführt, wo es im Wärmetausch mit der am Katalysatorbett frei werdenden Reaktionswärme auf die Reaktionstemperatur erwärmt wird.

Das bei der Umsetzung verbrauchte Methanol wird durch frisches Methanol 29 ersetzt, welches dem Pufferbehälter 3 zugeführt wird, in den auch das aus dem zweiten Verfahrensabschnitt zurückgeführte Methanol eingespeist wird. Aus dem Pufferbehälter 3 wird ein Methanolstrom 31 für die katalytische Umsetzung und ein Methanolstrom 30 als Waschmethanol für den zweiten Verfahrensabschnitt entnommen.

Der zweite Verfahrensabschnitt, der Gegenstand dieser Erfindung ist, ist also über die Stoffströme 22 (Schwefelwasserstoff-Kreisgas), 27 (Produktgasgemisch), 28 (abgetrenntes Waschmethanol) und 30 (frisches Waschmethanol) mit dem ersten Verfahrensabschnitt verbunden.

Das den ersten Verfahrensabschnitt verlassende Produktgasgemisch 27 hat in dem vorliegenden Beispiel folgende Eigenschaften:

| | |
|---|---|
| Methylmercaptan | 39 Gew.-% |
| Dimethylsulfid | 1,6 Gew.-% |
| Dimethylether | 2,7 Gew.-% |
| Inertgase (H₂, CO, CO₂, N₂) | 2,5 Gew.-% |
| Wasser | 15 Gew.-% |
| Schwefelwasserstoff | 34 Gew.-% |
| Methanol | 5 Gew.-% |
| Temperatur | 130°C |
| Druck | 8 bar |

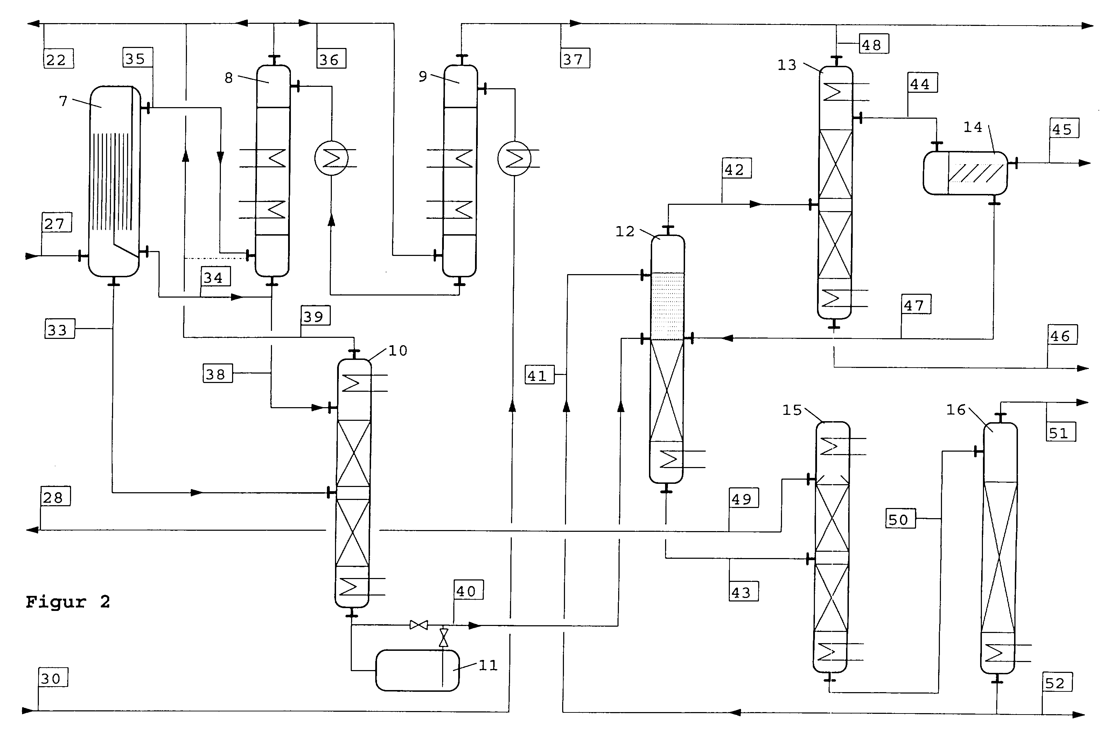

Eine konkrete Ausgestaltung des erfindungsgemäßen Verfahrens wird anhand des Verfahrensschemas von Figur 2 erläutert.

Das Produktgasgemisch mit den obigen Eigenschaften fällt mit einem Mengenstrom von 2,7 t/t MM (MM = Methylmercaptan) an und wird dem zweistufigen Partialkondensator 7 zugeführt.

In der ersten Kondensationsstufe werden vorwiegend die höher siedenden Anteile, also Methanol und Wasser, bei einer Temperatur von 60°C als wäßriges Kondensat 33 auskondensiert. Zur Kühlung kann normaltemperiertes Rückkühlwasser verwendet werden. In der zweiten Stufe wird ein organisches Kondensat 34, welches etwa 75 Gew.-% Methylmercaptan enthält, bei 25°C auskondensiert. Hierzu muß mit Kaltwasser von etwa 5°C gekühlt werden. Bei den hier betrachteten Mengenströmen ist eine Kälteleistung von etwa 180 kW/t MM erforderlich.

Der nicht kondensierte Restgasstrom 35 enthält neben Schwefelwasserstoff als Hauptkomponente noch etwa 30 % der im Produktgasstrom 27 vor Kondensation enthaltenen Menge Methylmercaptan. Das Methylmercaptan wird aus dem Restgasstrom durch Wäsche mit Methanol mehrstufig absorbiert. Dies geschieht im Methylmercaptan-Absorber 8 (MM-Absorber). Wegen der erheblichen Kondensations- beziehungsweise Absorptionswärme sind entlang der Kolonne Wärmeaustauschelemente angeordnet, um die Absorption isotherm bei einer Temperatur von bevorzugt 25°C durchführen zu können. Die abzuführende Absorptionswärme beträgt 48 kW/t MM. Auf diese Weise kann Methylmercaptan auf kleiner als 1 Vol.-% ausgewaschen werden. Als Kühlmedium kann Wasser mit einer Temperatur von 5°C verwendet werden.

Zur Absorption von Methylmercaptan wird der Restgasstrom dem MM-Absorber von unten zugeleitet und strömt im Gegenstrom zur Waschflüssigkeit nach oben. Auf den Kopf der Absorberkolonne wird Methanol als Waschflüssigkeit mit einer Temperatur von 15°C aufgegeben.

Der die Absorberkolonne verlassende Restgasstrom steht unter einem Druck von etwa 6 bar und weist neben Schwefelwasserstoff als Hauptkomponente 10 Vol.-% inerte Gase auf (H₂, CO₂, CO, N₂). Dieser Restgasstrom wird im Volumenverhältnis von 7 : 1 in einen Kreisgasstrom 22 und einen Ausschleusstrom 36 aufgeteilt. Das Kreisgas wird in den ersten Verfahrensabschnitt zurückgeführt und vor der zweiten Verdichtungsstufe bei einem Druck von 6 bar dem Schwefelwasserstoff-Frischgas zugemischt. Das Abzweigen des Ausschleusstromes ist notwendig, um eine Anreicherung der Inertgase im ersten Verfahrensabschnitt zu verhindern. Das Volumenverhältnis von 7 : 1 gewährleistet im vorliegenden Fall, daß die mit dem Schwefelwasserstoff-Frischgas eingeschleusten und im Synthesereaktor durch Methanolzersetzung gebildeten Mengen an Inertgasen kontinuierlich ausgeschleust werden.

Um Verluste von Schwefelwasserstoff zu vermeiden, wird der Ausschleusstrom erneut mit Methanol in einer Schwefelwasserstoff-Absorberkolonne (H₂S-Absorber) gewaschen. Bevorzugt wird auch diese Wäsche isotherm bei einer Temperatur von 25°C durchgeführt. Der Ausschleusstrom wird hierzu dem H₂S-Absorber von unten zugeleitet und strömt im Gegenstrom zur Waschflüssigkeit nach oben. Zur Durchführung der isothermen Absorption ist auch die H₂S-Absorberkolonne mit Kühlelementen ausgestattet, die die Absorptionswärme von etwa 18 kW/t MM abführen. Der den H₂S-Absorber verlassende gereinigte Ausschleusstrom 37 ist fast vollständig von Schwefelwasserstoff befreit (weniger als 0,1 Vol.-%) und kann der Verbrennung zugeleitet werden.

Für die erste Methanolwäsche im MM-Absorber und die zweite Methanolwäsche im H₂S-Absorber wird derselbe Methanolstrom verwendet. Hierzu wird Methanol aus dem Pufferbehälter 3 (siehe Figur 1) als Mengenstrom 30 entnommen und in einem Kühler auf 15°C abgekühlt, bevor er am Kopf des H₂S-Absorbers aufgegeben wird. Nach der Absorption von Schwefelwasserstoff im H₂S-Absorber ist das Waschmethanol mit etwa 15 Gew.-% Schwefelwasserstoff beladen. Es wird nach erneuter Kühlung auf 15°C am Kopf des MM-Absorbers zur Absorption des Methylmercaptans aus dem Restgasstrom aufgegeben. Im MM-Absorber belädt sich das Waschmethanol mit etwa 30 Gew.-% Methylmercaptan. Die Vorbeladung mit Schwefelwasserstoff wirkt sich nicht negativ auf die Absorption von Methylmercaptan aus.

Die beiden Kondensatströme 33 und 34 aus dem Partialkondensator sowie das Waschmethanol 38 enthalten große Mengen an gelöstem Schwefelwasserstoff (wäßriges Kondensat 33: ∼ 1 Gew.-%; organisches Kondensat 34: ∼ 20 Gew.-%; Waschmethanol 38: ∼ 19 Gew.-%). Zur Entfernung des Schwefelwasserstoffes aus diesen Stoffströmen wird eine Abtriebskolonne 10 mit 10 theoretischen Böden verwendet. Organisches Kondensat und Waschmethanol werden gemeinsam am Kopf der Kolonne aufgegeben, während das höher siedende wäßrige Kondensat etwa auf halber Höhe der Abtriebskolonne eingeführt wird. Die Destillation wird bei einem Druck von 8 bar durchgeführt. Bei diesem Druck beträgt die Siedetemperatur im Sumpf etwa 85°C.

Der Schwefelwasserstoff verläßt die Abtriebskolonne als Gasstrom 39 über Kopf und wird in den Kreisgasstrom 22 eingespeist. Der Gasstrom 39 enthält außer Schwefelwasserstoff noch Anteile der leicht siedenden Komponenten Methylmercaptan, Dimethylether und Dimethylsulfid. Um die Trennschärfe zu verbessern kann in den Kopf der Abtriebskolonne ein Partialkondensator integriert werden, der den Gehalt des Gasstromes 39 an Methylmercaptan bei einer gewählten Kondensationstemperatur von 25°C auf weniger als 10 Vol.-% beschränkt.

Alternativ zur Einspeisung in den Kreisgasstrom 22 kann der Gasstrom 39 auch mit dem Restgasstrom 35 zusammen in den MM-Absorber eingespeist werden (siehe gestrichelte Verbindung). Dadurch wird der Gehalt des Kreisgasstromes an Methylmercaptan vermindert. Bei direkter Einspeisung von Strom 39 in den Kreisgasstrom enthält dieser etwa 3 Vol.-% Methylmercaptan. Wird Strom 39 durch den MM-Absorber geleitet, kann der Gehalt des Kreisgasstromes an Methylmercaptan auf unter 1 Vol.-% vermindert werden.

Im Destillationssumpf der Abtriebskolonne fällt ein sogenanntes Rohprodukt an. Es weist etwa folgende Zusammensetzung auf:

| | |
|---|---|
| Methylmercaptan | 43 Gew.-% |
| Dimethylsulfid | 2 Gew.-% |
| Dimethylether | 2 Gew.-% |
| Methanol | 34,7 Gew.-% |
| Wasser | 18 Gew.-% |
| höhere Sulfide | < 0,5 Gew.-% |

Es wird nach Kühlung auf ca. 60°C im Pufferbehälter 11 gesammelt. Von hier aus wird es in das entsprechende Temperaturniveau einer Vortrennkolonne 12 eingespeist und dort in einen Methylmercaptan und Dimethylsulfid und in einen Methanol und Wasser enthaltenden Stoffstrom aufgetrennt. In der Vortrennkolonne wird ein extraktives Destillationsverfahren angewendet, um die im Rohprodukt vorliegenden azeotropen Gemische aufzutrennen. Als Extraktionsmittel wird Wasser verwendet, welches am Kopf der Vortrennkolonne aufgegeben wird. In der Vortrennkolonne herrscht ein Arbeitsdruck von 6 bar. Das Kopfprodukt der Vortrennkolonne enthält:

| | |
|---|---|
| Methylmercaptan | ∼ 93 Gew.-% |
| Dimethylsulfid | ∼ 4,5 Gew.-% |
| Dimethylether | ∼ 1,5 Gew.-% |
| Wasser | ∼ 1 Gew.-% |
| Methanol | Spuren |

Es wird bevorzugt ohne Zwischenkondensation als Mengenstrom 42 einer weiteren Destillation, der sogenannten Reinkolonne, zugeführt. Der dampfförmige Mengenstrom 42 wird etwa in die Mitte der Reinkolonne eingespeist, die ebenfalls mit einem Arbeitsdruck von 6 bar arbeitet. In der Reinkolonne erfolgt die Trennung von Methylmercaptan und Dimethylsulfid. Methylmercaptan fällt als Kopfprodukt an und wird im integrierten Kondensator kondensiert, indem zuerst der Rücklauf bei Siedetemperatur und dann die Produktentnahme durch Unterkühlung erzeugt wird. Das am Kopf der Reinkolonne austretende Kopfkondensat wird nach weiterer Kühlung auf 20°C im Wasserabscheider 14 in Methylmercaptan und Wasser getrennt. Zur Kompensation von Druckschwankungen wird der Reinkolonne ein Inertgas 48, bevorzugt Stickstoff, mit einem Druck von 6 bar überlagert.

Aus dem Wasserabscheider wird Methylmercaptan als Mengenstrom 45 in hoher Reinheit abgezogen. Es hat etwa folgende Zusammensetzung:

| | |
|---|---|
| Methylmercaptan | ∼ 98 Gew.-% |
| Dimethylsulfid | 0,1 Gew.-% |
| Dimethylether | 1,5 Gew.-% |
| Wasser | 0,37 Gew.-% |
| Methanol | 0,03 Gew.-% |
| Schwefelwasserstoff | Spuren |

Das abgetrennte Wasser kann als Mengenstrom 47 in die Vortrennkolonne eingespeist werden.

Das als Bodenprodukt der Reinkolonne anfallende Dimethylsulfid ist ebenfalls von hoher Reinheit und kann vermarktet werden. Der Mengenstrom 46 (Dimethylsulfid) beträgt etwa 2 Gew.-% vom Mengenstrom 45 (Methylmercaptan).

Der Destillationsrückstand der Vortrennkolonne hat etwa folgende Zusammensetzung:

| | |
|---|---|
| Methanol | ∼ 43 Gew.-% |
| Wasser | ∼ 54,5 Gew.-% |
| Sulfidische Nebenprodukte | ∼ 1 Gew.-% |
| Dimethylether | ∼ 1 Gew.-% |

Der Methanolanteil setzt sich zusammen aus dem Waschmethanol (Mengenstrom 30) (∼ 85 Gew.-%) und dem bei der katalytischen Synthese nicht vollständig umgesetzten Methanol. Der Wasseranteil entstammt dem Extraktionswasser (Mengenstrom 41) und dem bei der Synthese entstehenden Reaktionswasser. Die sulfidischen Nebenprodukte, hauptsächlich Dimethyldisulfid, werden ebenfalls bei der Synthese gebildet.

Zur Rückgewinnung des wertvollen Methanols wird der Destillationsrückstand aus der Vortrennkolonne nach Entspannung von 6 auf 1 bar einer Methanol/Wasser-Trennkolonne als Mengenstrom 43 zugeführt. Am Kopf der Kolonne wird Methanol abgezogen. Das Prozeßwasser fällt als Bodenprodukt an und enthält geringe Mengen Polysulfide.

In dieser Methanol/Wasser-Trennkolonne kann die Wasserkonzentration im Kopfprodukt und die Methanol-Konzentration im Sumpf jeweils auf 0,2 Gew.-% begrenzt werden.

Das am Kopf der Kolonne abgezogene Methanol wird als Mengenstrom 49 beziehungsweise 28 in den Pufferbehälter 3 (siehe Figur 1) zurückgegeben und damit der erneuten Verwendung als Waschmethanol und Synthesemethanol zugeführt. Es enthält maximal 0,5 Gew.-% Dimethyldisulfid.

Das als Bodenprodukt der Methanol/Wasser-Trennkolonne anfallende Prozeßwasser muß zu einem Teil, welcher dem bei der Synthese gebildeten Reaktionswasser entspricht, kontinuierlich aus dem Prozeß ausgeschleust werden. Der andere Teil, welcher etwa zwei Drittel der Menge des Prozeßwassers ausmacht, wird als Extraktionswasser 41 in die Vortrennkolonne eingespeist. Um eine Anreicherung von Polysulfiden im Extraktionswasser einerseits und die Geruchsbelastung des auszuschleusenden Reaktionswassers andererseits zu vermeiden, wird das Prozeßwasser auf den Kopf einer Abtriebssäule gegeben. Im Sumpf der Säule wird Dampf erzeugt und im Gegenstrom zum Prozeßwasser geführt. Aufgrund der heteroazeotropen Eigenschaften der Polysulfide werden diese mit dem Wasserdampf ausgeschleppt. Es werden nur etwa 5 % des Prozeßwassers als Dampfmenge benötigt, um ein vollständiges Austreiben der Polysulfide zu bewirken. Der mit den Polysulfiden angereicherte Kopfstrom der Abtriebssäule kann direkt zur Verbrennung gegeben werden.

Das die Abtriebssäule als Sumpfprodukt verlassende Prozeßwasser ist nahezu geruchsfrei.

## Patentansprüche

1. Verfahren zur Auftrennung des mit einer Temperatur von 100 bis 150°C und mit einem Druck von 6 bis 12 bar anfallenden Produktgasgemisches der katalytischen Synthese von Methylmercaptan aus Schwefelwasserstoff und Methanol in die Komponenten Methylmercaptan, Dimethylsulfid, Polysulfide, Wasser, Methanol, Schwefelwasserstoff und Inertgas enthaltend die folgenden Verfahrensschritte:
a) Auftrennen des Produktgasstromes in ein wäßriges, Methanol und Wasser enthaltendes, Kondensat und in ein organisches, Schwefelwasserstoff, Methylmercaptan und Dimethylsulfid enthaltendes, Kondensat sowie in einen Restgasstrom, welcher Schwefelwasserstoff und Methylmercaptan enthält, durch zweistufige Partialkondensation, wobei das wäßrige Kondensat bei Temperaturen zwischen 55 und 65°C und das organische Kondensat bei Temperaturen zwischen 15 und 30°C kondensiert wird,
b) Absorption von Methylmercaptan und Dimethylsulfid aus dem Restgasstrom in einer ersten Wäsche mit Methanol und Aufteilen des gewaschenen, schwefelwasserstoffreichen Gasstromes in einen Kreisgasstrom und einen Ausschleusstrom im Volumenverhältnis von 5 : 1 bis 20 : 1,
c) Absorption von Schwefelwasserstoff aus dem Ausschleusstrom in einer zweiten Wäsche mit Methanol und Entfernen des gereinigten Ausschleusstromes aus dem Verfahrensablauf, wobei für die zweite Wäsche frisches Methanol verwendet wird, welches nach Beladung mit Schwefelwasserstoff als Waschmittel für die erste Wäsche eingesetzt wird,
d) Destillieren des beladenen Waschmethanols sowie des wäßrigen und des organischen Kondensats zur Abtrennung von Schwefelwasserstoff als gasförmiges Kopfprodukt von den restlichen Komponenten des Produktgasgemisches, welche als flüssiges Rohprodukt im Destillationssumpf anfallen und Einspeisen des abgetrennten Schwefelwasserstoffes in den Kreisgasstrom oder in den Restgasstrom und
e) Auftrennen des Rohproduktes mittels weiterer Destillationen in die Komponenten Methylmercaptan, Dimethylsulfid, Dimethylether, Polysulfide, Methanol und Wasser.

2. Verfahren nach Anspruch 1,
**dadurch gekennzeichnet,**
daß die Absorption von Schwefelwasserstoff und Methylmercaptan durch Waschen mit Methanol isotherm bei einer Temperatur im Bereich zwischen 20 und 30°C vorgenommen wird.

3. Verfahren nach Anspruch 1,
**dadurch gekennzeichnet,**
daß das Rohprodukt, welches die azeotropen Gemische Methylmercaptan/Methanol und Dimethylsulfid/Methanol enthält, durch extraktive Destillation in einen Methylmercaptan und Dimethylsulfid und in einen Methanol und Wasser enthaltenden Stoffstrom, aufgespalten wird, wobei als Extraktionsmittel Wasser verwendet wird und Methylmercaptan und Dimethylsulfid als Kopfprodukt und Methanol und Wasser als Destillationsrückstand anfallen.

4. Verfahren nach Anspruch 3,
**dadurch gekennzeichnet,**
daß der Methylmercaptan und Dimethylsulfid enthaltende Stoffstrom destillativ in ein Methylmercaptan und Wasser enthaltendes Kopfkondensat und ein Dimethylsulfid enthaltendes Bodenprodukt aufgetrennt wird und daß das Kopfkondensat durch eine flüssig-flüssig Phasentrennung in Methylmercaptan hoher Reinheit und Wasser aufgespalten wird.

5. Verfahren nach einem der Ansprüche 3 oder 4,
**dadurch gekennzeichnet,**
daß der Methanol und Wasser enthaltende Destillationsrückstand einer weiteren Destillation zugeführt wird, wobei Methanol am Kopf kondensiert und zur Verwendung als Waschmethanol und für die Methylmercaptansynthese zurückgeführt wird und Wasser, welches noch geringe Menge Polysulfide enthält, als Bodenprodukt anfällt.

6. Verfahren nach Anspruch 5,
**dadurch gekennzeichnet,**
daß die im abgetrennten Wasser enthaltenen Polysulfide mittels Dampf ausgetrieben und einer Abgasverbrennung zugeführt werden.

7. Verfahren nach Anspruch 6,
**dadurch gekennzeichnet,**
daß das von den Polysulfiden gereinigte Wasser zu einem Teil, welcher dem im Syntheseprozeß gebildeten Reaktionswasser entspricht, aus dem Verfahrensablauf ausgeschleust und der restliche Teil als Extraktionswasser in die extraktive Destillation zurückgeführt wird.

## Claims

1. A process for separating the product gas mixture obtained during the catalytic synthesis of methyl mercaptan from hydrogen sulfide and methanol at a temperature of 100 to 150°C and a pressure of 6 to 12 bar into the components methyl mercaptan, dimethyl sulfide, polysulfides, water, methanol, hydrogen sulfide and inert gas, comprising the following process steps:
a) separating the product gas stream into an aqueous condensate which contains methanol and water and into an organic condensate which contains hydrogen sulfide, methyl mercaptan and dimethyl sulfide, and also into a residual gas stream which contains hydrogen sulfide and methyl mercaptan, by two-stage partial condensation, wherein the aqueous condensate is condensed at temperatures between 55 and 65°C and the organic condensate is condensed at temperatures between 15 and 30°C,
b) absorption of methyl mercaptan and dimethyl sulfide from the residual gas stream in a first scrubbing process using methanol and dividing the scrubbed, hydrogen sulfide-rich, gas stream into a circulating gas stream and an elimination stream in the ratio by volume of 5 : 1 to 20 : 1,
c) absorption of hydrogen sulfide from the elimination stream in a second scrubbing process using methanol and removing the purified elimination stream from the process, wherein fresh methanol is used for the second scrubbing process and this is used as the scrubbing agent in the first scrubbing process after being contaminated with hydrogen sulfide,
d) distilling the contaminated scrubbing methanol and the aqueous and organic condensate to separate hydrogen sulfide, as a gaseous head product, from the remaining components in the product gas mixture, which is obtained as a liquid crude product at the bottom of the distillation column and feeding the separated hydrogen sulfide into the circulating gas stream or into the residual gas stream and
e) separating the crude product, by means of further distillation, into the components methyl mercaptan, dimethyl sulfide, dimethyl ether, polysulfides, methanol and water.

2. A process according to Claim 1,
characterised in that
the absorption of hydrogen sulfide and methyl mercaptan by scrubbing with methanol is performed isothermally at a temperature in the range between 20 and 30°C.

3. A process according to Claim 1,
characterised in that
the crude product, which contains the azeotropic mixtures methyl mercaptan/methanol and dimethyl sulfide/methanol, is split into a substance stream containing methyl mercaptan and dimethyl sulfide and a substance stream containing methanol and water, by extractive distillation, wherein water is used as extraction agent and methyl mercaptan and dimethyl sulfide are obtained as the head product and methanol and water are obtained as the distillation residue.

4. A process according to Claim 3,
characterised in that
the substance stream containing methyl mercaptan and dimethyl sulfide is separated, by distillation, into a head condensate which contains methyl mercaptan and water and a bottom product which contains dimethyl sulfide and that the head condensate is split into high purity methyl mercaptan and water by means of liquid/liquid phase separation.

5. A process according to one of Claims 3 or 4,
characterised in that
the distillation residue containing methanol and water is supplied to another distillation process, wherein methanol is condensed at the head and is recycled for use as scrubbing methanol and for methyl mercaptan synthesis and water, which still contains small amounts of polysulfides, is obtained as the bottom product.

6. A process according to Claim 5,
characterised in that
the polysulfides contained in the separated water are driven out using steam and are supplied to a vent gas incineration process.

7. A process according to Claim 6,
characterised in that
some of the water from which the polysulfides have been removed, corresponding to the reaction water formed in the synthesis process, is removed from the process and the remainder is supplied as extraction water to the extractive distillation process.

## Revendications

1. Procédé de séparation du mélange gazeux, obtenu à une température de 100 à 150 °C et une pression de 6 à 12 bars, provenant de la synthèse catalytique du méthylmercaptan à partir d'acide sulfhydrique et de méthanol en composants méthylmercaptan, sulfure de diméthyle, polysulfure, eau, méthanol, acide sulfhydrique et gaz inerte, comprenant les étapes de procédé suivantes :
a) séparation du courant gazeux en un condensat aqueux contenant du méthanol et de l'eau et en condensat organique contenant de l'acide sulfhydrique, du méthylmercaptan et du sulfure de diméthyle, ainsi qu'en un courant gazeux résiduel qui contient de l'acide sulfhydrique et du méthylmercaptan, par une condensation partielle en deux étapes, le condensat aqueux étant condensé à des températures entre 55 et 65 °C et le condensat organique entre 15 et 30 °C,
b) absorption du méthylmercaptan et du sulfure de diméthyle du courant gazeux résiduel dans un premier lavage avec du méthanol et répartition du courant gazeux lavé, riche en acide sulfhydrique entre un courant gazeux en circulation et un courant évacué dans un rapport volumique de 5:1 à 20:1,
c) absorption de l'acide sulfhydrique du courant évacué dans un deuxième lavage avec du méthanol et élimination du courant d'évacuation purifié du processus, en utilisant pour le deuxième lavage du méthanol frais qui est utilisé, après avoir été chargé avec de l'acide sulfhydrique, comme agent de lavage pour le deuxième lavage,
d) distillation du méthanol de lavage chargé ainsi que du condensat aqueux et organique pour la séparation de l'acide sulfhydrique sous forme de produit de tête de distillation gazeux des autres composants du mélange gazeux, qui se trouvent sous forme de produit brut liquide dans le résidu de distillation et alimentation de l'acide sulfhydrique séparé dans le courant gazeux en circulation ou dans le courant de gaz résiduaires et
e) séparation du produit brut par des distillations ultérieures en composants méthylmercaptan, sulfure de diméthyle, diméthyléther, polysulfure, méthanol et eau.

2. Procédé selon la revendication 1, caractérisé en ce que l'absorption de l'acide sulfhydrique et du méthylmercaptan est réalisée par lavage avec du méthanol par voie isothermique à une température comprise dans la plage de 20 à 30 °C.

3. Procédé selon la revendication 1, caractérisé en ce que le produit brut, qui contient les mélanges azéotropiques méthylmercaptan/méthanol et sulfure de diméthyle/méthanol, est séparé par distillation extractive en un courant de substances contenant du méthylmercaptan et du sulfure de diméthyle et un courant de substances contenant du méthanol et de l'eau, en utilisant de l'eau comme agent d'extraction et le méthylmercaptan et le sulfure de diméthyle étant obtenus comme produit de tête de distillation et le méthanol et l'eau comme résidu de distillation.

4. Procédé selon la revendication 3, caractérisé en ce que le courant de substances contenant du méthylmercaptan et du sulfure de diméthyle est séparé par distillation en un condensat de tête contenant du méthylmercaptan et de l'eau et un fond de distillation contenant du sulfure de diméthyle et en ce que le condensat de tête est séparé par une séparation de phases liquide-liquide en méthylmercaptan d'une pureté élevée et en eau.

5. Procédé selon l'une des revendications 3 ou 4, caractérisé en ce que le résidu de distillation contenant du méthanol et de l'eau est alimenté dans une distillation ultérieure, le méthanol condensant en tête et étant recyclé pour être utilisé en tant que méthanol de lavage et pour la synthèse du méthylmercaptan et l'eau, contenant encore de faibles quantités de polysulfures, étant obtenue comme fond de distillation.

6. Procédé selon la revendication 5, caractérisé en ce que les polysulfures contenus dans l'eau séparée sont extraits à l'aide de vapeur et alimentés dans une installation de combustion des gaz résiduaires.

7. Procédé selon la revendication 6, caractérisé en ce qu'une partie de l'eau, correspondant à l'eau de réaction formée dans le processus de synthèse, purifiée des polysulfures, est évacuée du processus et en ce que la partie résiduelle est recyclée dans la distillation extractive comme eau d'extraction.
